# EUROPEAN PATENT SPECIFICATION

(11) **EP 4 078 169 B1**
(45) Date of publication and mention of the grant of the patent: **19.03.2025**
(21) Application number: 20823862.6
(22) Date of filing: 16.12.2020
(51) Int. Cl.: G01N 30/86, G01N 30/72, G01N 30/88

(54) **METHOD AND DEVICE FOR MULTIPLE TRANSITION MONITORING IN LC/MS**
VERFAHREN UND VORRICHTUNG ZUR MEHRFACHÜBERGANGSÜBERWACHUNG IN LC/MS
PROCÉDÉ ET DISPOSITIF DE SURVEILLANCE DE TRANSITION MULTIPLES EN LC/MS

(30) Priority: 17.12.2019 EP 19216968
(43) Date of publication of application: 26.10.2022
(73) Proprietor: Roche Diagnostics GmbH, 68305 Mannheim (DE); F. Hoffmann-La Roche AG, 4070 Basel (CH)
(72) Inventor: SCHWEINBERGER, Florian, 82377 Penzberg (DE); VAN DOORN, Aart Pieter, 82377 Penzberg (DE)
(74) Representative: Altmann Stößel Dick Patentanwälte PartG mbB
(86) International application number: PCT/EP2020/086476
(87) International publication number: WO 2021/122784

(56) References cited:
- WO-A1-2013/044401
- WO-A1-2018/116039
- WO-A2-2012/035412
- VINZENZ LANGE ET AL: "Selected reaction monitoring for quantitative proteomics: a tutorial", MOLECULAR SYSTEMS BIOLOGY, vol. 4, 14 October 2008 (2008-10-14), XP055033380, ISSN: 1744-4292, DOI: 10.1038/msb.2008.61
- MICHAEL S. BEREMAN ET AL: "The development of selected reaction monitoring methods for targeted proteomics via empirical refinement", PROTEOMICS, vol. 12, no. 8, 1 April 2012 (2012-04-01), DE, pages 1134 - 1141, XP055706222, ISSN: 1615-9853, DOI: 10.1002/pmic.201200042
- DAVID PRESSER: "Agilent 6400 Series Triple QuadrupoleLC/MS/MS Users Session; QQQ Method Development and Optimization", 13 October 2009 (2009-10-13), XP055193026, Retrieved from the Internet <URL:https://www.chem.agilent.com/Library/posters/Public/QQQ_Method_Development_Triple_and_Optimization.pdf> [retrieved on 20150602]
- LUDOVIC C. GILLET ET AL: "Targeted Data Extraction of the MS/MS Spectra Generated by Data-independent Acquisition: A New Concept for Consistent and Accurate Proteome Analysis", MOLECULAR & CELLULAR PROTEOMICS, vol. 11, no. 6, 12 June 2012 (2012-06-12), US, XP055471043, ISSN: 1535-9476, DOI: 10.1074/mcp.O111.016717
- MARTIN KRAUSS ET AL: "LC-high resolution MS in environmental analysis: from target screening to the identification of unknowns", ANALYTICAL AND BIOANALYTICAL CHEMISTRY, SPRINGER, BERLIN, DE, vol. 397, no. 3, 16 March 2010 (2010-03-16), pages 943 - 951, XP019839152, ISSN: 1618-2650
- CHRISTOPHER M. COLANGELO ET AL: "Review of software tools for design and analysis of large scale MRM proteomic datasets", METHODS, vol. 61, no. 3, 1 June 2013 (2013-06-01), NL, pages 287 - 298, XP055543136, ISSN: 1046-2023, DOI: 10.1016/j.ymeth.2013.05.004
- HAN BOMIE ET AL: "Proteomics: from hypothesis to quantitative assay on a single platform. Guidelines for developing MRM assays using ion trap mass spectrometers", BRIEFINGS IN FUNCTIONAL GENOMICS AND PROTEOMICS, HENRY STEWART, LONDON, GB, vol. 7, no. 5, 1 September 2008 (2008-09-01), pages 340 - 354, XP009128978, ISSN: 1473-9550, DOI: 10.1093/BFGP/ELN032

## Description

### Field of the invention

The invention relates to a method and a device for multiple transition monitoring using mass spectrometry techniques, specifically liquid chromatography and mass spectrometry.

### Related art

Multiple transition monitoring (MRM) during one liquid chromatography run reduces dwell times for each transition with increasing number of observer MRM. Known methods such as scheduled MRMs use only the times frames in a liquid chromatography run that is relevant to a certain transition. For example, analyte X with MRM Y is resulting in peak Z at time x and, thus, only a certain time of relevance such as x-15s to x+ 15 s may be recorded. Usually a certain time frame is defined with respect to a certain time before and after the peak maximum of the peak, usually referred to as the retention time.

US 2017/0328874 A1 describes a chromatograph mass spectrometer including: an MSn-1 analysis setter for setting an analysis execution period for performing an MSn-1 analysis, an execution time for the analysis and a loop time; an analysis period divider for dividing the analysis period into segments according to a change in number or analysis condition of MSn-1 analyses to be performed within the same time window; an MSn analysis setter for performing MSn-1 analysis to obtain mass spectrum data and for scheduling MSn analysis, an ion corresponding to a peak satisfying a set condition being designated as a precursor ion; an MSn analysis execution time allotter for allotting, in each segment, a time period for execution of the MSn analysis, the time period being calculated by subtracting an event execution time from the loop time; and an analysis executer for repeatedly performing MSn-1 analysis and MSn analysis in each segment.

WO 2017/216934 A1 describes an analysis schedule which is pre-created such that streams of a plurality of liquid chromatograms can operate in parallel and a mass spectrometer can collect data at the timing of each component elution. A control unit controls so as to: divide the time required to analyze each sample in a plurality of liquid chromatogram systems into pre-collection time, time during collection, and post-collection time; search and allocate time positions in which the time during collection in the liquid chromatogram units do not overlap; determine start times for the plurality of liquid chromatogram units to thereby create an analysis schedule; and thereafter perform analysis. The control unit further stores parameter sets for varying component elution times, adjusts analysis parameters so as to make data collection timings suitable for creating an analysis schedule, and changes the component elution times.

US 2015/0102219 A1 describes prior to multiple reaction monitoring measurement condition optimization, an analysis operator which prepares, for each precursor ion of an objective compound, two lists on a product-ion selection condition setting screen, i.e. a list which shows ions to be preferentially selected as product ions for which the optimization needs to be performed and a list which shows ions to be excluded from the optimization. When a measurement is performed, a product-ion scan measurement for the precursor ion of the objective compound is performed and a spectrum is obtained. Among the ions extracted from this spectrum, any ion registered in the excludable-ion list is excluded, while any ion registered in the preferred-ion list is preferentially selected as a product ion. For each combination of the m/z values of the precursor ion and the product ions thus determined, optimum conditions of the MRM measurement are searched for.

US 9,040,903 B2 describes mass spectrometry systems and methods which utilize a dynamic a data acquisition/instrument control methodology. These systems and methods employ artificial intelligence algorithms to greatly increase quantitative and/or identification accuracy during data acquisition. In an embodiment, the algorithms can adapt the instrument methods and systems during data acquisition to direct data acquisition resources to increase quantitative or identification accuracy of target analytes, such as proteins, peptides, and peptide fragments.

WO 2018/116039 A1 describes systems and methods for identifying actual XIC peaks of compounds of interest from samples. In one system, an actual XIC peak is identified using standard samples. The ratio of the quantity of the compound of interest in any two different samples is known, so this ratio is compared to the intensities of the XIC peak calculated in the two samples to identify an actual XIC peak. In another system, an actual XIC peak is identified using information about other compounds of interest in a plurality of samples. It is known that the XIC peaks of compounds of interest in the same samples have a similar distribution of retention times across those samples, so the distributions of retention times of XIC peaks are compared to identify actual XIC peaks.

JP 05835086 B2 describes creation of a mass calibration table. After a specimen is injected into a specimen vaporization chamber of GC, before the lapse of the time for completely eluting a specimen solvent from a column, mass analysis is not performed and data are not collected, either. Thus, mass calibration is prevented from being performed by erroneously utilizing an analysis result of the solvent. Thereafter, collection of data is started by scan measurement and when a signal strength of TIC becomes equal to or higher than a threshold value, it is recognized that the elution of components for mass calibration is started. Then, the scan measurement is executed while changing a scan speed, a mass spectrum of the different scan speed with respect to the component for mass calibration is acquired and based on the mass spectrums, a mass calibration table is created.

V. Lange et al. "Selected reaction monitoring for quantitative proteomics: a tutorial", MOLEC-ULAR SYSTEMS BIOLOGY, vol. 4, 14 October 2008, XP055033380, ISSN: 1744-4292, DOI: 10.1038/msb.2008.61 describes selected reaction monitoring (SRM) for reliable quantification of analytes of low abundance in complex mixtures. In an SRM experiment, a predefined precursor ion and one of its fragments are selected by the two mass filters of a triple quadrupole instrument and monitored over time for precise quantification. A series of transitions in combination with the retention time of the targeted peptide can constitute a definitive assay. Typically, a large number of peptides are quantified during a single LC-MS experiment. V. Lange et al. explain the application of SRM for quantitative proteomics, including the selection of proteotypic peptides and the optimization and validation of transitions.

Scheduled MRM in particular works well for very defined measurements and/or analyte mixtures with high concentrations. Despite the advantages of scheduled MRM, however, there is growing interest for maximizing column lifetimes, which may go hand in hand with significant peak shifts such that scheduled MRM with a fixed time frame for peak recording may lead to non reliable and incorrect results.

### Problem to be solved

It is therefore an objective of the present invention to provide a method and a device for multiple transition monitoring, which avoid the above-described disadvantages of known methods and devices. In particular, the method and the device shall allow reliable and correct multiple transition monitoring even in case of retention time shifts due to column aging, capillary exchanges, solvent composition inaccuracies, and other factors. Moreover, the method and the device shall allow lowering limit of quantification, specifically for critical analytes.

### Summary of the invention

This problem is solved by a method and a device for multiple transition monitoring, having the features of the independent claims. Preferred embodiments of the invention, which may be realized in an isolated way or in any arbitrary combination, are disclosed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, a method for multiple transition monitoring using a liquid chromatography mass spectrometry device is disclosed.

The term "multiple transition monitoring", also denoted multiple reaction monitoring (MRM), as used herein is a broad term and is to be given its ordinary and customary meaning to a person of ordinary skill in the art and is not to be limited to a special or customized meaning. The term specifically may refer, without limitation, to a method used in mass spectrometry, specifically in tandem mass spectrometry, in which multiple product ions from one or more precursor ions are monitored. As used herein, the term "monitored" is a broad term and is to be given its ordinary and customary meaning to a person of ordinary skill in the art and is not to be limited to a special or customized meaning. The term specifically may refer, without limitation, to determining and/or detecting of multiple product ions.

As used herein, the term "liquid chromatography mass spectrometry device" is a broad term and is to be given its ordinary and customary meaning to a person of ordinary skill in the art and is not to be limited to a special or customized meaning. The term specifically may refer, without limitation, to a combination of liquid chromatography with mass spectrometry. The liquid chromatography mass spectrometry device may be or may comprise at least one high-performance liquid chromatography (HPLC) device or at least one micro liquid chromatography (µLC) device. The liquid chromatography mass spectrometry device may comprise a liquid chromatography (LC) device and a mass spectrometry (MS) device, wherein the LC device and the MS are coupled via at least one interface.

As used herein, the term "liquid chromatography (LC) device" is a broad term and is to be given its ordinary and customary meaning to a person of ordinary skill in the art and is not to be limited to a special or customized meaning. The term specifically may refer, without limitation, to an analytical module configured to separate one or more analytes of interest of a sample from other components of the sample for detection of the one or more analytes with the mass spectrometry device. The LC device may comprise at least one LC column. For example, the LC device may be a single-column LC device or a multi-column LC device having a plurality of LC columns. The LC column may have a stationary phase through which a mobile phase is pumped in order to separate and/or elute and/or transfer the analytes of interest. The LC column may be exchangeable, for example after a predefined or pre-determined time and/or number of runs, and/or other suitable counters. For example, the LC column may be exchanged if one or more thresholds of one or more of a volume of solvent, a number of switching event of valves, a number of runs, a number of injections, a number of samples, a number of samples of a certain type, an LC pressure/curves are reached. As used herein, the term "mass spectrometry device" is a broad term and is to be given its ordinary and customary meaning to a person of ordinary skill in the art and is not to be limited to a special or customized meaning. The term specifically may refer, without limitation, to a mass analyzer configured for detecting at least one analyte based on mass to charge ratio. The mass spectrometry device may be or may comprise at least one quadrupole mass spectrometry device. The interface coupling the LC device and the MS may comprise at least one ionization source configured for generating of molecular ions and for transferring of the molecular ions into the gas phase.

As used herein, the term "sample" is a broad term and is to be given its ordinary and customary meaning to a person of ordinary skill in the art and is not to be limited to a special or customized meaning. The term specifically may refer, without limitation, to an arbitrary sample such as a biological sample, also called test sample, a quality control sample, an internal standard sample. The sample may comprise one or more analytes of interest. For example, the test sample may be selected from the group consisting of: a physiological fluid, including blood, serum, plasma, saliva, ocular lens fluid, cerebral spinal fluid, sweat, urine, milk, ascites fluid, mucous, synovial fluid, peritoneal fluid, amniotic fluid, tissue, cells or the like. The sample may be used directly as obtained from the respective source or may be subject of a pretreatment and/or sample preparation workflow. For example, the sample may be pretreated by adding an internal standard and/or by being diluted with another solution and/or by having being mixed with reagents or the like. For example, analytes of interest may be vitamin D, drugs of abuse, therapeutic drugs, hormones, and metabolites in general. The quality control sample may be a sample that mimics the test sample, and that contains known values of one or more quality control substances. The quality control substance may be identical to the analyte of interest or may be an analyte which generates by reaction or derivatization an analyte identical to the analyte of interest and/or may be an analyte of which the concentration is known and/or may be a substance which mimics the analyte of interest or that can be otherwise correlated to a certain analyte of interest. The internal standard sample may be a sample comprising at least one internal standard substance with a known concentration. For further details with respect to the sample, reference is made e.g. to EP 3 425 369 A1.

Other analytes of interest are possible.

The method comprises the following steps which, as an example, may be performed in the given order. It shall be noted, however, that a different order is also possible. Further, it is also possible to perform one or more of the method steps once or repeatedly. Further, it is possible to perform two or more of the method steps simultaneously or in a timely overlapping fashion. The method may comprise further method steps which are not listed.

The method comprises the following steps:
a) determining at least one data set from at least one data base, the data set comprising at least one reference measurement of at least one transition of at least one analyte with the liquid chromatography mass spectrometry device;
b) determining at least one reference peak information of the transition of the analyte using an initial setting of a measurement window, wherein the measurement window is defined by a time frame of retention times;
c) determining an actual setting of the measurement window considering the reference peak information, wherein the determining comprises adjusting the time frame;
d) measuring the transition of the analyte with the liquid chromatography mass spectrometry device and determining a measured peak information of the transition of the analyte using the actual setting of the measurement window.

As used herein, the term "data set" is a broad term and is to be given its ordinary and customary meaning to a person of ordinary skill in the art and is not to be limited to a special or customized meaning. The term specifically may refer, without limitation, to stored and/or deposited information about at least one previous MRM measurement such as from previous runs. The information about the previous MRM measurement may comprise at least one chromatogram and/or at least one information evaluated from the chromatogram such as peak maximum, retention time, peak start time, peak end time, peak width, in particular the full width half maximum, peak shape, tailing factor, and/or any type of peak fitting and filtering. The tailing factor T may be determined by T= W_{0.05}/(2d), wherein W_{0.05} is the peak width at 0.05 of the peak height and d is a distance between a perpendicular line through the peak maximum and a leading edge of the peak at 0.05 of the peak height. As used herein, the term "data base" is a broad term and is to be given its ordinary and customary meaning to a person of ordinary skill in the art and is not to be limited to a special or customized meaning. The term specifically may refer, without limitation, to a collection of data comprising the at least one data set. The data base may comprise at least one table and/or at least one look-up table in which the at least one data set is stored. The data base may comprise at least one storage unit configured to store the data set. As used herein, the term "determining at least one data set from at least one data base" is a broad term and is to be given its ordinary and customary meaning to a person of ordinary skill in the art and is not to be limited to a special or customized meaning. The term specifically may refer, without limitation, to access the data base and to retrieve the data set from the data base.

As used herein, the term "reference measurement" is a broad term and is to be given its ordinary and customary meaning to a person of ordinary skill in the art and is not to be limited to a special or customized meaning. The term specifically may refer, without limitation, to at least one previous MRM measurement such as a MRM measurement of a previous run and/or at least one prediction about a MRM measurement. The reference measurement may comprise or may be at least one known MRM transition. The reference measurement may be at least one measurement of at least one quality control sample acquired during a previous quality control run and/or at least one measurement of at least one internal standard sample acquired during a previous internal standard sample run and/or at least one measurement of the test sample acquired during a previous run. The reference measurement may be at least one measurement acquired and/or determined and/or measured in the same way and under the same or at least similar and/or comparable conditions as the measurement of the actual test sample. The reference measurements and the measuring of the transition of the analyte may be performed under substantially the same conditions. For example, the reference measurement and the measuring of the transition of the analyte may be performed under constant chromatographic conditions, specifically with the same LC column and eluents. The method may apply to known compounds and well-known conditions. The method may however also comprise predicting at least one reference measurement, specifically in case of changes of gradients. The predicting may comprise considering aging of LC column, capillary exchanges, solvent composition inaccuracies, and other factors.

As used herein, the term "reference peak information" is a broad term and is to be given its ordinary and customary meaning to a person of ordinary skill in the art and is not to be limited to a special or customized meaning. The term specifically may refer, without limitation, to at least one information of a peak, i.e. a local maximum, of the chromatogram corresponding and/or relating to the analyte of interest of the reference measurement which is suitable to limit the relevant time frame for measurement of the analyte of interest. The reference peak information may comprise one or more of: peak maximum, retention time, peak start time, peak end time, peak width, in particular the full width half maximum, peak shape, tailing factor and/or any type of peak fitting and filtering. The determining of the reference peak information may comprise evaluating the reference measurement. The evaluating may comprise performing at least one data analysis comprising performing at least one peak finding algorithm and/or performing at least one peak fitting algorithm. The evaluating may comprise one or more of checking of raw data, preprocessing, smoothening, background reduction or removal, peak detection, peak integration.

As used herein, the term "measurement window" is a broad term and is to be given its ordinary and customary meaning to a person of ordinary skill in the art and is not to be limited to a special or customized meaning. The term specifically may refer, without limitation, to a time frame in which the measurement of the analyte of interest is performed. The measurement window is defined by a time frame of retention times. Limiting the measurement of the analyte of interest to a certain pre-defined time frame is generally known. As used herein, the term "setting" of the measurement window is a broad term and is to be given its ordinary and customary meaning to a person of ordinary skill in the art and is not to be limited to a special or customized meaning. The term specifically may refer, without limitation, to values for one or both limits of the measurement window. Specifically, the setting of the measurement window may comprise one or both of a value for a lower limit of the measurement window, i.e. a retention time at which the measurement starts, and a value for an upper limit of the measurement window, i.e. a retention time at which the measurement stops.

The term "initial setting" is a broad term and is to be given its ordinary and customary meaning to a person of ordinary skill in the art and is not to be limited to a special or customized meaning. The term specifically may refer, without limitation, to a setting of the measurement window which is used for determining the reference peak information. The initial setting may be pre-determined and/or pre-defined. For example, in case of a first measurement after change of a column of the LC device, the initial setting may be a default setting which may be deposited in the data base. The method may comprise at least one initial calibration step, wherein in the initial calibration step the reference measurement and/or the initial setting may be determined. The initial calibration step may be performed during and/or subsequent to at least one quality control run of the liquid chromatography mass spectrometry device and/or during and/or subsequent to at least one internal standard samples run. A start of the initial calibration step may be triggered by changing of a column of the liquid chromatography mass spectrometry device and/or after a predefined or pre-determined time and/or after a predefined or predetermined number of runs, or other suitable counters. The term "trigger" as used herein, may refer to either an automatic procedure that is initiated and executed automatically or a warning generated for and prompting a user to manually set the setting to the initial setting. In case of performing the method repeatedly, the initial setting may be a setting of the measurement window determined from the measurement of at least one prior run or a plurality of prior runs, such as a mean value for the limits of the time frame.

The term "actual setting" is a broad term and is to be given its ordinary and customary meaning to a person of ordinary skill in the art and is not to be limited to a special or customized meaning. The term specifically may refer, without limitation, to a setting of the measurement window determined by considering the reference peak information determined in step b). In addition, to considering the reference peak information of only the preceding measurement, the actual setting may be determined considering the preceding measurement or a plurality of preceding measurements. The determining of the actual setting may comprise evaluating the reference peak information and thereby determining a lower and/or an upper limit of the measurement window. Specifically, at least one automated analysis of retention times may be performed. Moreover, the measurement window may be automatically reassigned. The actual setting may be determined and/or calculated based on expected retention time and tailing factor. The actual setting may be determined by comparing peak, in particular signal intensity, and background. For example, signal intensity and background may be compared by defining and/or using at least one threshold value at which the peak starts and/or at least one threshold value at which the peak ends. The actual setting may be determined by making a prediction based on a plurality of datasets.

The initial setting may comprise a broader time frame of retention times compared to the actual setting and/or the actual setting may be shifted in retention time compared to the initial setting. In the latter case the width of the measurement window may be maintained. Specifically, the initial setting of the measurement window may be selected so broad such that it is ensured that the peak corresponding to the analyte of interest lies within the time frame. Subsequently the initial setting of the measurement window may be optimized in view of measurement results which allows for reducing width of the measurement window and/or for positioning the measurement window, specifically to take into account changes of the LC column such as due to aging or other changes. The reference measurement may comprise or may be a known MRM transition, wherein the method comprises optimizing their measurement. The term "determining the actual setting" is a broad term and is to be given its ordinary and customary meaning to a person of ordinary skill in the art and is not to be limited to a special or customized meaning. The term specifically may refer, without limitation, to adapting and/or changing the initial setting of the measurement window depending on at least one subsequent measurement. The timing of the measurement may be fixed. However, due to variation of the peak position as a result of ongoing analysis, specifically in case of repeatedly performing method steps a) to d), the retention time may shift and may be adapted based on prior measurements. The method may comprise adjustment of the MRM scheduled timing as a function of changing parameters over time. Thus, the optimized retention time may be used to optimize scheduled MRM measurements and thus freeing up time for more MRM transitions.

The method comprises in step d) measuring the transition of the analyte within a sample with the liquid chromatography mass spectrometry device. The measurement may be triggered by a user, e.g. by entering at least one input to at least one human-machine-interface of the liquid chromatography mass spectrometry device.

The actual setting of the measurement window is used for determining the measured peak information of the transition of the analyte. As used herein, the term "measured peak information" is a broad term and is to be given its ordinary and customary meaning to a person of ordinary skill in the art and is not to be limited to a special or customized meaning. The term specifically may refer, without limitation, to at least one information of a peak of the chromatogram measured in step d) corresponding and/or relating to the analyte of interest using the actual setting of the measurement window. The measured peak information comprises one or more of: peak maximum, retention time, peak start time, peak end time, peak width, in particular the full width half maximum, peak shape, tailing factor. The determining of the measured peak information may comprise evaluating the actual measurement. The evaluating may comprise performing at least one data analysis comprising performing at least one peak finding algorithm and/or performing at least one peak fitting algorithm.

The method further may comprise updating the data set by adding the measurement of the transition of the analyte to the data set. Specifically, in case the method steps a) to d) are performed repeatedly, the data set may be updated after performing step d) such that the subsequent step a) is performed using an updated initial setting. Thus, the updating may be performed permanently.

Method steps a) to d) may be performed repeatedly. In step a) the most recent measurement may be used as reference measurement. In step b) the reference peak information of the transition of the analyte may be determined using the most recently determined actual setting as initial setting. After a plurality of repetitions of methods steps a) to d), in step a) a mean value of a plurality of preceding measurements may be used as reference measurement. The mean value may be determined by using at least five preceding measurements. Additionally or alternatively, in particular in case of larger changes between runs, a moving average of a plurality of preceding measurements may be used as reference measurement. Additionally or alternatively, a maximum amount of preceding measurements for calculating the mean value may be limited. Using more than one measurement may ensure that the reference data is corrected with respect to outliers or sample influence.

A width of the measurement window may narrow with number of repetitions. Thus, the measurement window becomes even better and/or more beneficial for clearing up more MS detection window. The detection window may be a time frame in which the mass spectrometry device has to perform a measurement of a sample. Specifically, the detection window may be the maximum time possible between two consecutive sample inputs in the LC column.

The method may further comprise at least one *in-situ* adjustment step. The *in-situ* adjustment step may be performed during step d). In the *in-situ* adjustment step intensity of the transition of the analyte may be monitored during the measurement and compared to at least one predetermined or predefined threshold level. The liquid chromatography mass spectrometry device may comprise at least one further data base configured for storing at least one definition of threshold levels and/or threshold values. For example, the at least one threshold level and/or the at least one threshold value may be defined by percentual change of signal intensity to background. Additionally or alternatively, at least one absolute value may be used as threshold for determining exceedance or undershoot. The further data base may be configured to receive input information from the data base such as values for the threshold levels. Thus, the threshold levels may be data driven. If the intensity falls below the predefined threshold level acquisition of the transition may be stopped. The pre-determined or predefined threshold level may be defined by a factor X times the signal-to-noise ratio which is also known from the start. The *in-situ* adjustment step may be implemented as a feedback loop with automated live adjustment of measurement parameter. For example, a measurement, i.e. a specific MRM, may start at a time known on the basis of previous measurements. A run time of the measurement may be determined on the basis of an actual measured intensity of this MRM. In case the intensity falls below the predetermined or predefined threshold level the acquisition of that MRM may be stopped and may allow to free dwell time for other MRMs running at the same time. This approach may be reliable for well articulated peaks. However, problems may arise when signal to noise is low, e.g. a small peak with high variation in individual signal. For these cases it may be advantageous that the predetermined or predefined threshold level may be given by an end of a fit curve such as a Gaussian curve. The fit curve may allow defining the peak region such as start of the peak and end of the peak. In particular, a certain peak height at the end of the peak, denoted as end of the fit curve, may be used as threshold level. In principle, use of a fit curve during measurement is problematic since the full signal is not present at this stage. However, a fit curve such as a Gaussian curve can be used since the fit parameters of the Gaussian or other fit curve may be determined from the one or more preceding measurements. This may allow determining all parameters of the fit curve with only a single iteration. Additionally or alternatively, the fit curve may be determined from a measurement of an internal standard. This may allow accelerating the fitting procedure. Additionally or alternatively, a combination of fit results of different peaks of the same analyte may be used to enhance robustness of the fit result. The fit curve, in particular of a Gaussian, may be independent or less dependent from background and, thus, advantageous even at high noise.

The method steps b) to c) and in step d) the determining of the measured peak information of the transition of the analyte may be performed by at least one computer. Specifically, the method steps b) to c) and in step d) the determining of the measured peak information of the transition of the analyte may be performed fully automatically. The method specifically may fully or partially be computer-implemented, specifically on a computer of a device for multiple transition monitoring, such as a processor.

The method may comprise compensating for column aging and/or for further impacts such as capillary exchanges, solvent composition inaccuracies, and the like.

In a further aspect, a computer program including computer-executable instructions for performing the method according to any one of the embodiments as described herein is disclosed, specifically method steps a) to c) and determining of the measured peak information in step d), when the program is executed on a computer or computer network, specifically a processor of the device for multiple transition monitoring.

Thus, generally speaking, disclosed and proposed herein is a computer program including computer-executable instructions for performing the method according to the present invention in one or more of the embodiments enclosed herein when the program is executed on a computer or computer network. Specifically, the computer program may be stored on a computer-readable data carrier. Thus, specifically, one, more than one or even all of the method steps as indicated above may be performed by using a computer or a computer network, preferably by using a computer program. The computer specifically may be fully or partially integrated into the device for multiple transition monitoring, and the computer programs specifically may be embodied as a software. Alternatively, however, at least part of the computer may also be located outside the device for multiple transition monitoring.

Further disclosed and proposed herein is a computer program product having program code means, in order to perform the method according to the present invention in one or more of the embodiments enclosed herein when the program is executed on a computer or computer network, e.g. one or more of the method steps mentioned above. Specifically, the program code means may be stored on a computer-readable data carrier.

Further disclosed and proposed herein is a data carrier having a data structure stored thereon, which, after loading into a computer or computer network, such as into a working memory or main memory of the computer or computer network, may execute the method according to one or more of the embodiments disclosed herein, specifically one or more of the method steps mentioned above.

Further disclosed and proposed herein is a computer program product with program code means stored on a machine-readable carrier, in order to perform the method according to one or more of the embodiments disclosed herein, when the program is executed on a computer or computer network, specifically one or more of the method steps mentioned above. As used herein, a computer program product refers to the program as a tradable product. The product may generally exist in an arbitrary format, such as in a paper format, or on a computer-readable data carrier. Specifically, the computer program product may be distributed over a data network.

Finally, disclosed and proposed herein is a modulated data signal which contains instructions readable by a computer system or computer network, for performing the method according to one or more of the embodiments disclosed herein, specifically one or more of the method steps mentioned above.

Specifically, further disclosed herein are:
- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

In a further aspect of the present invention, a device for multiple transition monitoring of at least one analyte in a sample is disclosed. The device comprises
- at least one liquid chromatography mass spectrometer device configured for multiple transition monitoring;
- at least one data base configured for storing at least one data set comprising at least one reference measurement of at least one transition of at least one analyte;
- at least one evaluation device, wherein the evaluation device is configured for determining at least one reference peak information of the transition of the analyte using an initial setting of a measurement window, wherein the measurement window is defined by a time frame of retention times, wherein the evaluation device is configured for determining an actual setting of the measurement window considering the reference peak information, wherein the determining comprises adjusting the time frame, wherein the evaluation device is configured for determining a measured peak information of the transition of the analyte of a subsequent measurement of the transition of the analyte with the liquid chromatography mass spectrometry device using the actual setting of the measurement window.

The device may be configured to perform the method according to any one of the preceding embodiments. For most of the terms used herein and possible definitions, reference may be made to the description of the methods above.

As further used herein, the term "evaluation device" generally refers to an arbitrary device adapted to perform the method steps as described above, preferably by using at least one data processing device and, more preferably, by using at least one processor and/or at least one application-specific integrated circuit. Thus, as an example, the at least one evaluation device may comprise at least one data processing device having a software code stored thereon comprising a number of computer commands. The evaluation device may provide one or more hardware elements for performing one or more of the named operations and/or may provide one or more processors with software running thereon for performing one or more of the method steps.

The methods and devices according to the present invention may provide a large number of advantages over known methods and devices for multiple transition monitoring. Thus, specifically, dynamic MRMs on the basis of previous runs may free up valuable channel time and consequently increase sampling rate or time for analytes. This is particularly beneficial for analytes that should be measured with high sensitivity. Influences that may be caused by changes of components over their lifetime, e.g. column aging, and/or further impacts such as capillary exchanges, solvent composition inaccuracies, and other factors, and resulting retention time shifts or changes and impact on spray stabilization, can be compensated within the boundary of the available time window. Deviations arising from column batch to batch variations can be compensated, too. The method does not require hardware changes but can be implemented as fast and powerful in situ algorithms.

Summarizing and without excluding further possible embodiments, the following embodiments may be envisaged:
Embodiment 1: Method for multiple transition monitoring using a liquid chromatography mass spectrometry device, the method comprises the following steps:
   a) determining at least one data set from at least one data base, the data set comprising at least one reference measurement of at least one transition of at least one analyte with the liquid chromatography mass spectrometry device;
   b) determining at least one reference peak information of the transition of the analyte using an initial setting of a measurement window, wherein the measurement window is defined by a time frame of retention times;
   c) determining an actual setting of the measurement window considering the reference peak information, wherein the determining comprises adjusting the time frame;
   d) measuring the transition of the analyte with the liquid chromatography mass spectrometry device and determining a measured peak information of the transition of the analyte using the actual setting of the measurement window.
Embodiment 2: The method according to the preceding embodiment, wherein the method further comprises updating the data set by adding the measurement of the transition of the analyte to the data set.
Embodiment 3: The method according to the preceding embodiment, wherein method steps a) to d) are performed repeatedly, wherein in step a) the most recent measurement is used as reference measurement, wherein in step b) the reference peak information of the transition of the analyte is determined using the most recently determined actual setting as initial setting.
Embodiment 4: The method according to the pre-preceding embodiment, wherein in step a) a mean value of a plurality of preceding measurements is used as reference measurement.
Embodiment 5: The method according to the preceding embodiment, wherein the mean value is determined by using at least five preceding measurements.
Embodiment 6: The method according to any one of the two preceding embodiments, wherein a maximum amount of preceding measurements for calculating the mean value is limited.
Embodiment 7: The method according to embodiment 2, wherein in step a) a moving average of a plurality of preceding measurements is used as reference measurement.
Embodiment 8: The method according to any one of the preceding embodiments, wherein the method further comprises at least one *in-situ* adjustment step, wherein the *in-situ* adjustment step is performed during step d), wherein in the *in-situ* adjustment step intensity of the transition of the analyte is monitored during the measurement and compared to at least one predetermined or predefined threshold level, wherein if the intensity falls below the predefined threshold level acquisition of the transition is stopped.
Embodiment 9: The method according to the preceding embodiment, wherein the at least one threshold level and/or the at least one threshold value is defined by percentual change of signal intensity to background and/or at least one absolute value is used as threshold for determining exceedance or undershoot.
Embodiment 10: The method according to the pre-preceding embodiment, wherein the predetermined or predefined threshold level is given by an end of a fit curve such as a Gaussian curve, wherein the parameters of the fit curve are determined from one or more preceding measurements, and/or wherein the fit curve is determined from a measurement of an internal standard, and/or wherein a combination of fit results of different peaks of the same analyte are used to enhance robustness of the fit result.
Embodiment 11: The method according to any one of the preceding embodiments, wherein the reference peak information and/or the measured peak information comprise one or more of: peak maximum, retention time, peak start time, peak end time, peak width, in particular the full width half maximum, peak shape, tailing factor, and/or any type of peak fitting and filtering.
Embodiment 12: The method according to any one of the preceding embodiments, wherein the method comprises at least one initial calibration step, wherein in the initial calibration step the reference measurement and/or the initial setting are determined.
Embodiment 13: The method according to the preceding embodiment, wherein the initial calibration step is performed during and/or subsequent to at least one quality control run of the liquid chromatography mass spectrometry device and/or during and/or subsequent to at least one internal standard samples run.
Embodiment 14: The method according to any one of the two preceding embodiments, wherein a start of the initial calibration step is triggered by changing of a column of the liquid chromatography mass spectrometry device and/or after a predefined or pre-determined time and/or after a predefined or predetermined number of runs, or other suitable counters.
Embodiment 15: The method according to any one of the preceding embodiments, wherein the initial setting comprises a broad time frame of retention times compared to the actual setting.
Embodiment 16: The method according to any one of the preceding embodiments, wherein the method comprises repeating method steps a) to d), wherein a width of the measurement window narrows with number of repetitions.
Embodiment 17: The method according to any one of the preceding embodiments, wherein the reference measurements and the measuring of the transition of the analyte are performed under substantially the same conditions.
Embodiment 18: The method according to any one of the preceding embodiments, wherein the method steps b) to c) and in step d) the determining of the measured peak information of the transition of the analyte are performed by at least one computer.
Embodiment 19: The method according to any one of the preceding embodiments, wherein the method comprises compensating for column aging and/or for further impacts such as capillary exchanges, solvent composition inaccuracies, and the like.
Embodiment 20: The method according to any one of the preceding embodiments, wherein the actual setting is determined and/or calculated based on expected retention time and tailing factor, and/or wherein the actual setting is determined by comparing peak and background, and/or wherein the actual setting is determined by making a prediction based on a plurality of datasets.
Embodiment 21: A device for multiple transition monitoring of at least one analyte in a sample comprising:
   - at least one liquid chromatography mass spectrometer device configured for multiple transition monitoring;
   - at least one data base configured for storing at least one data set comprising at least one reference measurement of at least one transition of at least one analyte;
   - at least one evaluation device, wherein the evaluation device is configured for determining at least one reference peak information of the transition of the analyte using an initial setting of a measurement window, wherein the measurement window is defined by a time frame of retention times, wherein the evaluation device is configured for determining an actual setting of the measurement window considering the reference peak information, wherein the determining comprises adjusting the time frame, wherein the evaluation device is configured for determining a measured peak information of the transition of the analyte of a subsequent measurement of the transition of the analyte with the liquid chromatography mass spectrometry device using the actual setting of the measurement window.
Embodiment 22: The device according to the preceding embodiment, wherein the device is configured to perform the method according to any one of the preceding embodiments referring to a method.

### Short description of the figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figures 1A and B: show a flow chart of a method according to the present invention;
- Figures 2A and B: show a comparison of a usual approach (Figure 2A) and the method according to the present invention (Figure 2B);
- Figures 3A and B: show a further comparison of measurement windows of a usual approach (Figure 3A) and of the method according to the present invention (Figure 3B); and
- Figure 4: shows a device for multiple transition monitoring according to the present invention.

### Exemplary embodiments

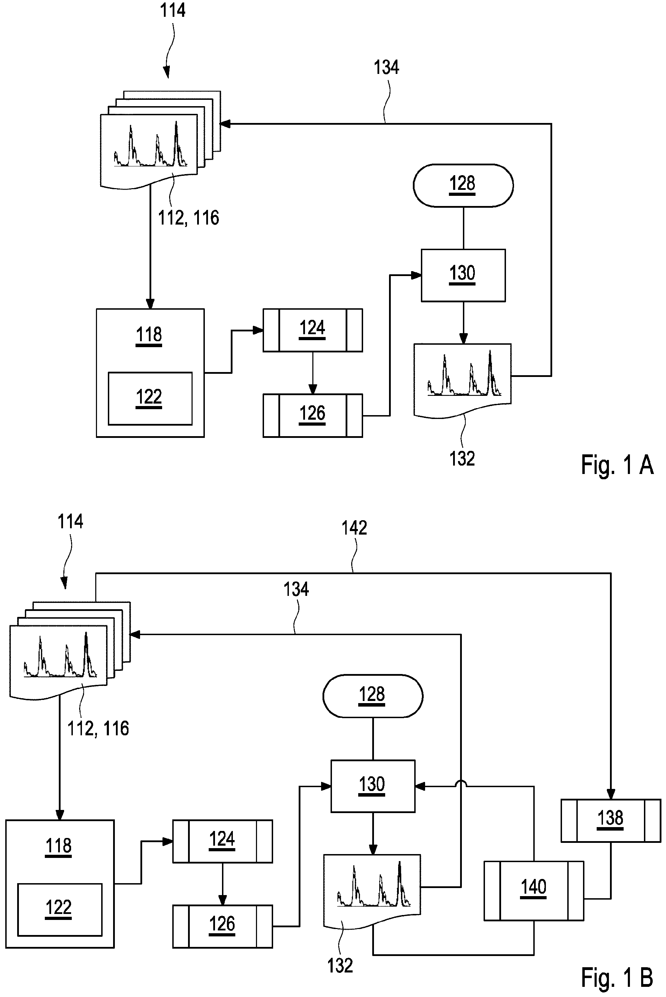

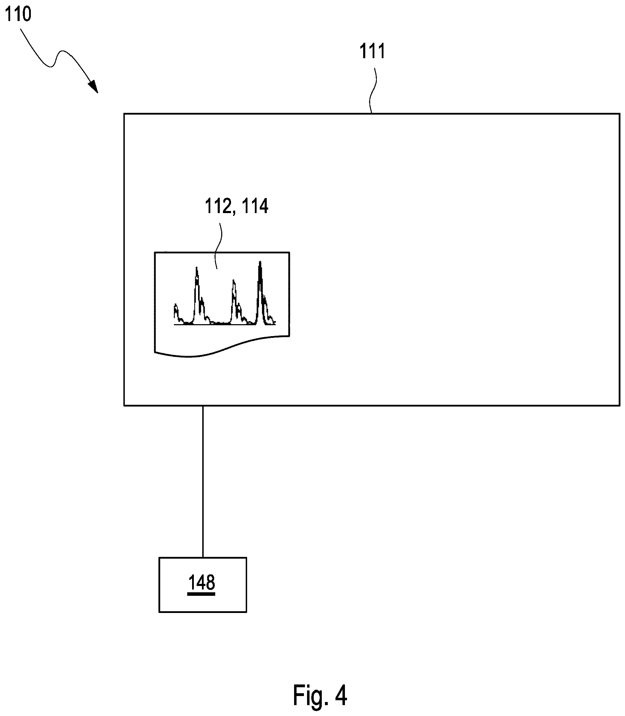

Figure 1A shows a flow chart of a method for multiple transition monitoring using a liquid chromatography mass spectrometry device 111, an embodiment of which is shown in Figure 4, according to the present invention.

The method comprises in step a) determining at least one data set 112 from at least one data base 114. The data set 112 comprises at least one reference measurement 116 of at least one transition of at least one analyte with the liquid chromatography mass spectrometry device 111. The data set 112 may be and/or may comprise stored and/or deposited information about at least one previous MRM measurement such as from previous runs. The information about the previous MRM measurement may comprise at least one chromatogram and/or at least one information evaluated from the chromatogram such as peak maximum, retention time, peak start time, peak end time, peak width, in particular the full width half maximum, peak shape, tailing factor, and/or any type of peak fitting and filtering. The data base 114 may comprise at least one table and/or at least one look-up table in which the at least one data set 112 is stored. The data base may comprise at least one storage unit configured to store the data set.

The reference measurement may comprise or may be at least one known MRM transition. The reference measurement may be at least one measurement of at least one quality control sample acquired during a previous quality control run and/or at least one measurement of at least one internal standard sample acquired during a previous internal standard sample run and/or at least one measurement of the test sample acquired during a previous run. The reference measurement may be at least one measurement acquired and/or determined and/or measured in the same way and under the same or at least similar and/or comparable conditions as the measurement of the actual test sample. The reference measurements and the measuring of the transition of the analyte may be performed under substantially the same conditions. For example, the reference measurement and the measuring of the transition of the analyte may be performed under constant chromatographic conditions, specifically with the same LC column and eluent. The method may apply to known compounds and well-known conditions. The method may comprise predicting at least one reference measurement, specifically in case of changes of gradients. The predicting may comprise considering aging of LC column, capillary exchanges, solvent composition inaccuracies, and other factors.

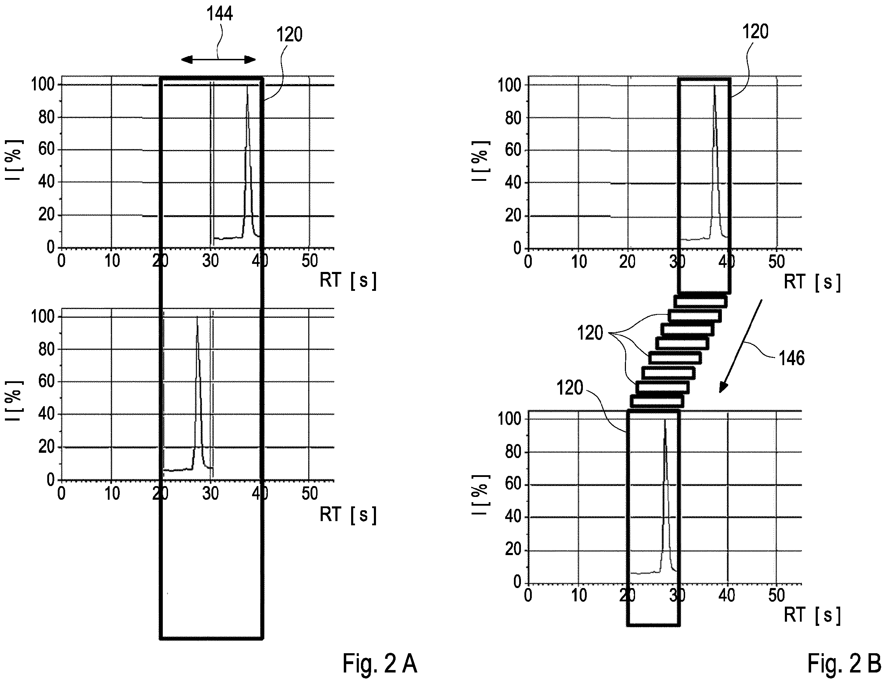

The method comprises in step b) (denoted with reference number 118) determining at least one reference peak information of the transition of the analyte using an initial setting of a measurement window 120, wherein the measurement window 120 is defined by a time frame of retention times. Examples of measurement windows 120 are shown in Figure 2B. The reference peak information may be at least one information of a peak, i.e. a local maximum, of the chromatogram corresponding and/or relating to the analyte of interest of the reference measurement which is suitable to limit the relevant time frame for measurement of the analyte of interest. The reference peak information may comprise one or more of: peak maximum, retention time, peak start time, peak end time, peak width, in particular the full width half maximum, peak shape, tailing factor, and/or any type of peak fitting and filtering. The determining of the reference peak information may comprise evaluating 122 the reference measurement. The evaluating 122 may comprise performing at least one data analysis comprising performing at least one peak finding algorithm and/or performing at least one peak fitting algorithm. The evaluating may comprise one or more of checking of raw data, preprocessing, smoothening, background reduction or removal, peak detection, peak integration.

The measurement window 120 is defined by a time frame of retention times. Limiting the measurement of the analyte of interest to a certain pre-defined time frame is generally known. The setting of the measurement window 120 may comprise values for one or both limits of the measurement window 120. Specifically, the setting of the measurement window 120 may comprise one or both of a value for a lower limit of the measurement window, i.e. a retention time at which the measurement starts, and a value for an upper limit of the measurement window, i.e. a retention time at which the measurement stops.

The initial setting may be a setting of the measurement window 120 which is used for determining the reference peak information. The initial setting may be pre-determined and/or pre-defined. For example, in case of a first measurement after change of a column of the LC device, the initial setting may be a default setting which may be deposited in the data base. The method may comprise at least one initial calibration step, not shown here, wherein in the initial calibration step the reference measurement and/or the initial setting may be determined. The initial calibration step may be performed during and/or subsequent to at least one quality control run of the liquid chromatography mass spectrometry device and/or during and/or subsequent to at least one internal standard samples run. A start of the initial calibration step may be triggered by changing of a column of the liquid chromatography mass spectrometry device 111 and/or after a predefined or pre-determined time and/or after a predefined or predetermined number of runs, or other suitable counters. In case of performing the method repeatedly, the initial setting may be a setting of the measurement window 120 determined from the measurement of at least one prior run or a plurality of prior runs, such as a mean value for the limits of the time frame.

The method furthermore comprises in step c) determining an actual setting of the measurement window 120 considering the reference peak information, wherein the determining comprises adjusting the time frame. The actual setting may be a setting of the measurement window 120 determined by considering the reference peak information determined in step b). In addition, to considering the reference peak information of only the preceding measurement, the actual setting may be determined considering the preceding measurement or a plurality of preceding measurements. The determining of the actual setting may comprise evaluating the reference peak information and thereby determining a lower and/or an upper limit of the measurement window. Specifically, at least one automated analysis of retention times may be performed, denoted with reference number 124. Moreover, the measurement window may be automatically reassigned, denoted with reference number 126. The actual setting may be determined and/or calculated based on expected retention time and tailing factor. The actual setting may be determined by comparing peak, in particular signal intensity, and background. For example, signal intensity and background may be compared by defining and/or using at least one threshold value at which the peak starts and/or at least one threshold value at which the peak ends. The actual setting may be determined by making a prediction based on a plurality of datasets.

The initial setting may comprise a broader time frame of retention times compared to the actual setting. Specifically, the initial setting of the measurement window 120 may be selected so broad such that it is ensured that the peak corresponding to the analyte of interest lies within the time frame. Subsequently the initial setting of the measurement window 120 may be optimized in view of measurement results which allows for reducing width of the measurement window and/or for positioning the measurement window 120, specifically to take into account changes of the LC column such as due to aging or other changes. The reference measurement may comprise or may be a known MRM transition, wherein the method comprises optimizing their measurement. The determining of the actual setting may comprise adapting and/or changing the initial setting of the measurement window depending on at least one subsequent measurement. The timing of the measurement may be fixed. However, due to variation of the peak position as a result of ongoing analysis, specifically in case of repeatedly performing method steps a) to d), the retention time may shift and may be adapted based on prior measurements. The method may comprise adjustment of the MRM scheduled timing as a function of changing parameters over time. Thus, the optimized retention time may be used to optimize scheduled MRM measurements and thus freeing up time for more MRM transitions.

The method comprises in step d) measuring, denoted with reference number 130, the transition of the analyte within a sample with the liquid chromatography mass spectrometry device. The measurement may be triggered 128 by a user e.g. by entering at least one input to at least one human-machine-interface of the liquid chromatography mass spectrometry device 111.

The method further comprises in step d) determining a measured peak information, denoted with reference number 132, of the transition of the analyte using the actual setting of the measurement window 120. The measured peak information may be at least one information of a peak of the chromatogram measured in step d) corresponding and/or relating to the analyte of interest using the actual setting of the measurement window 120. The measured peak information comprises one or more of: peak maximum, retention time, peak start time, peak end time, peak width, in particular the full width half maximum, peak shape, tailing factor, and/or any type of peak fitting and filtering. The determining of the measured peak information may comprise evaluating the actual measurement. The evaluating may comprise performing at least one data analysis comprising performing at least one peak finding algorithm and/or performing at least one peak fitting algorithm.

The method further may comprise updating 134 the data set 112 by adding the measurement of the transition of the analyte to the data set 112. Specifically, in case the method steps a) to d) are performed repeatedly, the data set 112 may be updated after performing step d) such that the subsequent step a) is performed using an updated initial setting. Thus, the updating 134 may be performed permanently.

Method steps a) to d) may be performed repeatedly. In step a) the most recent measurement may be used as reference measurement. In step b) the reference peak information of the transition of the analyte may be determined using the most recently determined actual setting as initial setting. After a plurality of repetitions of methods steps a) to d), in step a) a mean value of a plurality of preceding measurements may be used as reference measurement. The mean value may be determined by using at least five preceding measurements. Additionally or alternatively, in particular in case of larger changes between runs, a moving average of a plurality of preceding measurements may be used as reference measurement. Additionally or alternatively, a maximum amount of preceding measurements for calculating the mean value may be limited. Using more than one measurement may ensure that the reference data is corrected with respect to outliers or sample influence.

A width of the measurement window 120 may narrow with number of repetitions. Thus, the measurement window 120 becomes even better and/or more beneficial for clearing up more MS detection window. The detection window may be a time frame in which the mass spectrometry device has to perform a measurement of a sample.

Figure 1B shows a further flowchart of the method according to the present invention, wherein, in addition to the embodiment shown in Figure 1A, the method may further comprise at least one in-situ adjustment step 136. The *in-situ* adjustment step 136 may be performed during step d). In the *in-situ* adjustment step 136 intensity of the transition of the analyte may be monitored during the measurement and compared to at least one predetermined or predefined threshold level. The liquid chromatography mass spectrometry device 111 may comprise at least one further data base 138 configured for storing at least one definition of threshold levels and/or threshold values. For example, the at least one threshold level and/or the at least one threshold value may be defined by percentual change of signal intensity to background. Additionally or alternatively, at least one absolute value may be used as threshold for determining exceedance or undershoot. The further data base 138 may be configured to receive (denoted with reference number 142) input information from the data base 114 such as values for the threshold levels. Thus, the threshold levels may be data driven. If the intensity falls below the predefined threshold level acquisition of the transition may be stopped. The predetermined or predefined threshold level may be defined by a factor X times the signal-to-noise ratio which is also known from the start. The *in-situ* adjustment step may be implemented as a feedback loop 140 with automated live adjustment of measurement parameter. For example, a measurement, i.e. a specific MRM, may start at a time known on the basis of previous measurements. A run time of the measurement may be determined on the basis of an actual measured intensity of this MRM. In case the intensity falls below the predetermined or predefined threshold level the acquisition of that MRM may be stopped and may allow to free dwell time for other MRMs running at the same time. This approach may be reliable for well articulated peaks. However, problems may arise when signal to noise is low, e.g. a small peak with high variation in individual signal. For these cases it may be advantageous that the predetermined or predefined threshold level may be given by an end of fit curve such as a Gaussian curve. The fit curve may allow defining the peak region such as start of the peak and end of the peak. In particular, a certain peak height at the end of the peak, denoted as end of the fit curve, may be used as threshold level. In principle, use of a fit curve during measurement is problematic since the full signal is not present at this stage. However, a fit curve such as a Gaussian curve can be used since the parameters of the Gaussian or other fit curve may be determined from the one or more preceding measurements. This may allow determining all parameters of the fit curve with only a single iteration. Additionally or alternatively, the fit curve may be determined from a measurement of an internal standard. This may allow accelerating the fitting procedure. Additionally or alternatively, a combination of fit results of different peaks of the same analyte may be used to enhance robustness of the fit result. The fit curve, in particular of a Gaussian, may be independent or less dependent from background and, thus, advantageous even at high noise.

The method steps b) to c) and in step d) the determining of the measured peak information of the transition of the analyte may be performed by at least one computer. Specifically, the method steps b) to c) and in step d) the determining of the measured peak information of the transition of the analyte may be performed fully automatically. The method specifically may fully or partially be computer-implemented, specifically on a computer of a device for multiple transition monitoring, such as a processor.

Figures 2A and B show a comparison of a usual approach, shown in Figure 2A, and the method according to the present invention, shown in Figure 2B, such as described with respect to Figures 1A and 1B. Specifically, intensity I in % as a function of retention time RT in seconds is depicted. In Figures 2A and 2B, the upper plot shows the chromatogram for a new LC column and/or initial conditions and the lower plot shows the chromatogram for an aged LC column and/or potential other changes in the experimental performance of the system. For the usual approach shown in Figure 2A large measurement windows are used in order to compensate potential changes (denoted with arrow 144) over lifetime of the LC column. For example, the width Δt of the measurement window may be Δt =20 s and the position of the measurement window may be from 20 to 40 s. In Figure 2B the measurement window 120 can be selected narrower compared to Figure 2A, e.g. Δt =10 s. Moreover, the measurement window 120 can be maintained constant or can be even narrowed due to repeating method steps a) to d) and permanent reassignment (denoted with arrow 146) of the measurement window 120 based on prior measurements and *in-situ* adjustment.

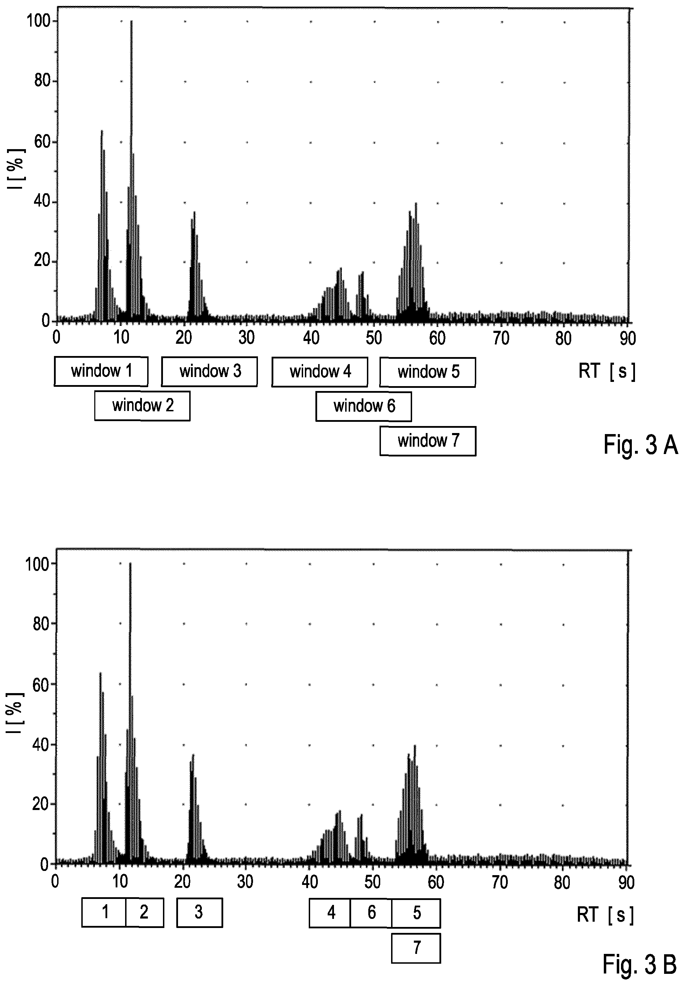

Figures 3A and B show a further comparison of measurement windows of a usual approach, shown in Figure 3A and of the method according to the present invention, shown in Figure 3B. Specifically, intensity I in % as a function of retention time RT in seconds is depicted. Moreover, in Figures 3A and 3B seven measurement windows and their width are shown each corresponding to a respective peak in the chromatogram. For the usual approach shown in Figure 3A large measurement windows are used in order to compensate potential changes over lifetime of the LC column. In contrast, in Figure 3B the measurement windows 120 are narrowed compared to Figure 3A. Using such narrow measurement windows is possible due to permanent reassignment of the measurement window 120 based on prior measurements and *in-situ* adjustment. No safety margins are necessary. The measurements windows show less overlap. Higher sampling rate per peak is possible resulting in more points per peak.

Figure 4 shows highly schematically a device 110 for multiple transition monitoring according to the present invention. The device 110 comprises the at least one liquid chromatography mass spectrometer device 111 configured for multiple transition monitoring. The liquid chromatography mass spectrometry device 111 may be or may comprise at least one high-performance liquid chromatography (HPLC) device or at least one micro liquid chromatography (µLC) device. The liquid chromatography mass spectrometry device 111 may comprise a liquid chromatography (LC) device and a mass spectrometry (MS) device, wherein the LC device and the MS are coupled via at least one interface. The LC device may comprise at least one LC column. For example, the LC device may be a single-column LC device or a multi-column LC device having a plurality of LC columns. The LC column may have a stationary phase through which a mobile phase is pumped in order to separate and/or elute and/or transfer the analytes of interest. The LC column may be exchangeable, for example after a predefined or pre-determined time and/or number of runs, and/or other suitable counters. The mass spectrometry device may be or may comprise at least one quadrupole mass spectrometry device. The interface coupling the LC device and the MS may comprise at least one ionization source configured for generating of molecular ions and for transferring of the molecular ions into the gas phase.

The device 110 further comprises the data base 114 configured for storing the data set 112 comprising the at least one reference measurement 116 of at least one transition of at least one analyte. With respect to description of the data set 112 and data base 114 reference is made to the description of Figures 1A and 1B above.

The device 110 comprises at least one evaluation device 148. The evaluation device 148 is configured for determining at least one reference peak information of the transition of the analyte using an initial setting of the measurement window 120. The measurement window 120 is defined by a time frame of retention times. The evaluation device 148 is configured for determining an actual setting of the measurement window 120 considering the reference peak information. The determining comprises adjusting the time frame. The evaluation device 148 is configured for determining a measured peak information of the transition of the analyte of a subsequent measurement of the transition of the analyte with the liquid chromatography mass spectrometry device 111 using the actual setting of the measurement window 120.

### List of reference numbers

- 110: device for multiple transition monitoring
- 111: CMS device
- 112: data set
- 114: data base
- 116: reference measurement
- 118: step b)
- 120: measurement window
- 122: evaluating the reference measurement
- 124: automated analysis
- 126: reassigning measurement window
- 128: trigger
- 130: measurement transition
- 132: determining measured peak information
- 134: updating
- 136: in-situ adjustment step
- 138: further data base
- 140: feedback loop
- 142: receiving input
- 144: arrow
- 146: arrow
- 148: evaluation device

## Claims

1. Method for multiple transition monitoring using a liquid chromatography mass spectrometry device (111), the method comprises the following steps:
a) determining at least one data set (112) from at least one data base (114), the data set (112) comprising at least one reference measurement (116) of at least one transition of at least one analyte with the liquid chromatography mass spectrometry device (111);
b) determining at least one reference peak information of the transition of the analyte using an initial setting of a measurement window (120), wherein the measurement window (120) is defined by a time frame of retention times;
c) determining an actual setting of the measurement window (120) considering the reference peak information, wherein the determining comprises adjusting the time frame, wherein the actual setting is a setting of the measurement window (120) determined by considering the reference peak information determined in step b);
d) measuring the transition of the analyte with the liquid chromatography mass spectrometry device (111) and determining a measured peak information of the transition of the analyte using the actual setting of the measurement window (120),
said method being **characterized in that** in step a) a mean value and/or a moving average of a plurality of preceding measurements is used as reference measurement (116).

2. The method according to the preceding claim, wherein the method further comprises updating (124) the data set (112) by adding the measurement of the transition of the analyte to the data set (112).

3. The method according to the preceding claim, wherein method steps a) to d) are performed repeatedly, wherein in step a) the most recent measurement is used as reference measurement (116), wherein in step b) the reference peak information of the transition of the analyte is determined using the most recently determined actual setting as initial setting.

4. The method according to any one of the preceding claims, wherein the method further comprises at least one *in-situ* adjustment step (136), wherein the *in-situ* adjustment step (136) is performed during step d), wherein in the *in-situ* adjustment step (136) intensity of the transition of the analyte is monitored during the measurement and compared to at least one predetermined or predefined threshold level, wherein if the intensity falls below the threshold level acquisition of the transition is stopped.

5. The method according to any one of the preceding claims, wherein the reference peak information and/or the measured peak information comprise one or more of: peak maximum, retention time, peak start time, peak end time, peak width, in particular the full width half maximum, peak shape, tailing factor, and/or any type of peak fitting and filtering.

6. The method according to any one of the preceding claims, wherein the method comprises at least one initial calibration step, wherein in the initial calibration step the reference measurement and/or the initial setting are determined.

7. The method according to the preceding claim, wherein the initial calibration step is performed during and/or subsequent to at least one quality control run of the liquid chromatography mass spectrometry device (111) and/or during and/or subsequent to at least one internal standard samples run.

8. The method according to any one of the two preceding claims, wherein a start of the initial calibration step is triggered by changing of a column of the liquid chromatography mass spectrometry device (111) and/or after a predefined or pre-determined time and/or after a predefined or predetermined number of runs, or other suitable counters.

9. The method according to any one of the preceding claims, wherein the initial setting comprises a broad time frame of retention times compared to the actual setting.

10. The method according to any one of the preceding claims, wherein the method comprises repeating method steps a) to d), wherein a width of the measurement window (120) narrows with number of repetitions.

11. The method according to any one of the preceding claims, wherein the reference measurements and the measuring of the transition of the analyte are performed under substantially the same conditions.

12. The method according to any one of the preceding claims, wherein the method steps b) to c) and in step d) the determining of the measured peak information of the transition of the analyte are performed by at least one computer.

13. A device (110) for multiple transition monitoring of at least one analyte in a sample comprising:
- at least one liquid chromatography mass spectrometer device (111) configured for multiple transition monitoring;
- at least one data base (112) configured for storing at least one data set (114) comprising at least one reference measurement (116) of at least one transition of at least one analyte;
- at least one evaluation device (148), wherein the evaluation device (148) is configured for determining at least one reference peak information of the transition of the analyte using an initial setting of a measurement window (120), wherein the measurement window (120) is defined by a time frame of retention times, wherein the evaluation device (148) is configured for determining an actual setting of the measurement window (120) considering the reference peak information, wherein the determining comprises adjusting the time frame, wherein the actual setting is a setting of the measurement window (120) determined by considering the determined reference peak information, wherein the evaluation device (148) is configured for determining a measured peak information of the transition of the analyte of a subsequent measurement of the transition of the analyte with the liquid chromatography mass spectrometry device (111) using the actual setting of the measurement window (120),
said device being **characterized in that** a mean value and/or a moving average of a plurality of preceding measurements is used as reference measurement (116).

14. The device (110) according to the preceding claim, wherein the device (110) is configured to perform the method according to any one of the preceding claims referring to a method.

## Patentansprüche

1. Verfahren zur Mehrfachübergangsüberwachung unter Verwendung einer Flüssigchromatographie-Massenspektrometrie-Vorrichtung (111), wobei das Verfahren die folgenden Schritte umfasst:
a) Bestimmen mindestens eines Datensatzes (112) aus mindestens einer Datenbank (114), wobei der Datensatz (112) mindestens eine Referenzmessung (116) mindestens eines Übergangs mindestens eines Analyten mit der Flüssigchromatographie-Massenspektrometrie-Vorrichtung (111) umfasst;
b) Bestimmen mindestens einer Referenzpeakinformation des Übergangs des Analyten unter Verwendung einer Ausgangseinstellung eines Messfensters (120), wobei das Messfenster (120) durch einen Zeitrahmen von Retentionszeiten definiert wird;
c) Bestimmen einer Isteinstellung des Messfensters (120) unter Berücksichtigung der Referenzpeakinformation, wobei das Bestimmen das Einstellen des Zeitrahmens umfasst, wobei die Isteinstellung eine Einstellung des Messfensters (120) ist, die unter Berücksichtigung der in Schritt b) bestimmten Referenzpeakinformation bestimmt wurde;
d) Messen des Übergangs des Analyten mit der Flüssigchromatographie-Massenspektrometrie-Vorrichtung (111) und Bestimmen einer gemessenen Peakinformation des Übergangs des Analyten unter Verwendung der Isteinstellung des Messfensters (120),
wobei das Verfahren **dadurch gekennzeichnet ist, dass** in Schritt a) ein Mittelwert und/oder ein gleitender Durchschnitt einer Vielzahl von vorangegangenen Messungen als Referenzmessung (116) verwendet wird.

2. Verfahren nach dem vorstehenden Anspruch, wobei das Verfahren ferner das Aktualisieren (124) des Datensatzes (112) durch Hinzufügen der Messung des Übergangs des Analyten zu dem Datensatz (112) umfasst.

3. Verfahren nach dem vorstehenden Anspruch, wobei die Verfahrensschritte a) bis d) wiederholt durchgeführt werden, wobei in Schritt a) die letzte Messung als Referenzmessung (116) verwendet wird, wobei in Schritt b) die Referenzpeakinformation des Übergangs des Analyten unter Verwendung der zuletzt bestimmten Isteinstellung als Ausgangseinstellung bestimmt wird.

4. Verfahren nach einem der vorstehenden Ansprüche, wobei das Verfahren ferner mindestens einen In-situ-Einstellungsschritt (136) umfasst, wobei der In-situ-Einstellungsschritt (136) während Schritt d) durchgeführt wird, wobei in dem In-situ-Einstellungsschritt (136) die Intensität des Übergangs des Analyten während der Messung überwacht und mit mindestens einem vorbestimmten oder vordefinierten Schwellenniveau verglichen wird, wobei, wenn die Intensität unter das Schwellenniveau fällt, die Erfassung des Übergangs gestoppt wird.

5. Verfahren nach einem der vorstehenden Ansprüche, wobei die Referenzpeakinformation und/oder die gemessene Peakinformation eines oder mehreres von Folgendem umfassen/umfasst: Peakmaximum, Retentionszeit, Peakstartzeit, Peakendzeit, Peakbreite, insbesondere die Halbwertsbreite, Peakform, Tailing-Faktor und/oder jede Art von Peakanpassen und -filtern.

6. Verfahren nach einem der vorstehenden Ansprüche, wobei das Verfahren mindestens einen Ausgangskalibrierungsschritt umfasst, wobei in dem Ausgangskalibrierungsschritt die Referenzmessung und/oder die Ausgangseinstellung bestimmt werden/wird.

7. Verfahren nach dem vorstehenden Anspruch, wobei der Ausgangskalibrierungsschritt während und/oder nach mindestens einem Qualitätskontrolldurchlauf der Flüssigchromatographie-Massenspektrometrie-Vorrichtung (111) und/oder während und/oder nach mindestens einem Durchlauf mit internen Standardproben durchgeführt wird.

8. Verfahren nach einem der beiden vorstehenden Ansprüche, wobei ein Start des Ausgangskalibrierungsschrittes durch Wechseln einer Säule der Flüssigchromatographie-Massenspektrometrie-Vorrichtung (111) und/oder nach einer vordefinierten oder vorbestimmten Zeit und/oder nach einer vordefinierten oder vorbestimmten Anzahl von Durchläufen oder anderen geeigneten Zählern ausgelöst wird.

9. Verfahren nach einem der vorstehenden Ansprüche, wobei die Ausgangseinstellung im Vergleich zu der Isteinstellung einen breiten Zeitrahmen von Retentionszeiten umfasst.

10. Verfahren nach einem der vorstehenden Ansprüche, wobei das Verfahren das Wiederholen der Verfahrensschritte a) bis d) umfasst, wobei eine Breite des Messfensters (120) mit der Anzahl an Wiederholungen enger wird.

11. Verfahren nach einem der vorstehenden Ansprüche, wobei die Referenzmessungen und das Messen des Übergangs des Analyten unter im Wesentlichen denselben Bedingungen vorgenommen werden.

12. Verfahren nach einem der vorstehenden Ansprüche, wobei die Verfahrensschritte b) bis c) und in Schritt d) das Bestimmen der gemessenen Peakinformation des Übergangs des Analyten von mindestens einem Computer ausgeführt werden.

13. Vorrichtung (110) zur Mehrfachübergangsüberwachung mindestens eines Analyten in einer Probe, umfassend:
- mindestens eine Flüssigchromatographie-Massenspektrometrie-Vorrichtung (111), die für Mehrfachübergangsüberwachung ausgebildet ist;
- mindestens eine Datenbank (112), die zum Speichern mindestens eines Datensatzes (114) ausgebildet ist, der mindestens eine Referenzmessung (116) mindestens eines Übergangs mindestens eines Analyten umfasst;
- mindestens eine Auswertevorrichtung (148), wobei die Auswertevorrichtung (148) zum Bestimmen mindestens einer Referenzpeakinformation des Übergangs des Analyten unter Verwendung einer Ausgangseinstellung eines Messfensters (120) ausgebildet ist, wobei das Messfenster (120) durch einen Zeitrahmen von Retentionszeiten definiert wird, wobei die Auswertevorrichtung (148) zum Bestimmen einer Isteinstellung des Messfensters (120) unter Berücksichtigung der Referenzpeakinformation ausgebildet ist, wobei das Bestimmen das Einstellen des Zeitrahmens umfasst, wobei die Isteinstellung eine Einstellung des Messfensters (120) ist, die unter Berücksichtigung der bestimmten Referenzpeakinformation bestimmt wurde, wobei die Auswertevorrichtung (148) zum Bestimmen einer gemessenen Peakinformation des Übergangs des Analyten einer Folgemessung des Übergangs des Analyten mit der Flüssigchromatographie-Massenspektrometrie-Vorrichtung (111) unter Verwendung der Isteinstellung des Messfensters (120) ausgebildet ist, wobei die Vorrichtung **dadurch gekennzeichnet ist, dass** ein Mittelwert und/oder ein gleitender Durchschnitt einer Vielzahl von vorangegangenen Messungen als Referenzmessung (116) verwendet werden/wird.

14. Vorrichtung (110) nach dem vorstehenden Anspruch, wobei die Vorrichtung (110) zum Ausführen des Verfahrens nach einem der vorstehenden Ansprüche, die sich auf ein Verfahren beziehen, ausgebildet ist.

## Revendications

1. Procédé de surveillance de transitions multiples en utilisant un dispositif de chromatographie liquide-spectrométrie de masse (111), le procédé comprend les étapes suivantes :
a) détermination d'au moins un ensemble de données (112) à partir d'au moins une base de données (114), l'ensemble de données (112) comprenant au moins une mesure de référence (116) d'au moins une transition d'au moins un analyte avec le dispositif de chromatographie liquide-spectrométrie de masse (111) ;
b) détermination d'au moins une information de pic de référence de la transition de l'analyte en utilisant un réglage initial d'une fenêtre de mesure (120), la fenêtre de mesure (120) étant définie par un intervalle temporel de temps de rétention ;
c) détermination d'un réglage réel de la fenêtre de mesure (120) prenant en compte l'information de pic de référence, la détermination comprenant l'ajustement de l'intervalle temporel, le réglage réel étant un réglage de la fenêtre de mesure (120) déterminé en prenant en compte l'information de pic de référence déterminée dans l'étape b) ;
d) mesure de la transition de l'analyte avec le dispositif de chromatographie liquide-spectrométrie de masse (111) et détermination d'une information de pic mesuré de la transition de l'analyte en utilisant le réglage réel de la fenêtre de mesure (120),
ledit procédé étant **caractérisé en ce que** dans l'étape a) une valeur moyenne et/ou une moyenne mobile d'une pluralité de mesures précédentes est utilisée en tant que mesure de référence (116).

2. Procédé selon la revendication précédente, dans lequel le procédé comprend en outre la mise à jour (124) de l'ensemble de données (112) en ajoutant la mesure de la transition de l'analyte à l'ensemble de données (112).

3. Procédé selon la revendication précédente, dans lequel les étapes a) à d) du procédé sont réalisées de manière répétée, dans lequel dans l'étape a) la mesure la plus récente est utilisée comme mesure de référence (116), dans lequel dans l'étape b) l'information de pic de référence de la transition de l'analyte est déterminée en utilisant le réglage réel le plus récemment déterminé en tant que réglage initial.

4. Procédé selon l'une quelconque des revendications précédentes, dans lequel le procédé comprend en outre au moins une étape d'ajustement in situ (136), dans lequel l'étape d'ajustement in situ (136) est réalisée pendant l'étape d), dans lequel dans l'étape d'ajustement in situ (136) l'intensité de la transition de l'analyte est surveillée pendant la mesure et comparée à au moins un niveau de seuil prédéterminé ou prédéfini, dans lequel si l'intensité chute en dessous du niveau de seuil l'acquisition de la transition est arrêtée.

5. Procédé selon l'une quelconque des revendications précédentes, dans lequel l'information de pic de référence et/ou l'information de pic mesuré comprennent un ou plusieurs parmi : le maximum du pic, le temps de rétention, le temps de début du pic, le temps de fin du pic, la largeur du pic, en particulier la largeur à mi-hauteur, la forme du pic, le facteur de traînée et/ou n'importe quel type de réglage et de filtrage du pic.

6. Procédé selon l'une quelconque des revendications précédentes, dans lequel le procédé comprend au moins une étape d'étalonnage initial, dans lequel dans l'étape d'étalonnage initial la mesure de référence et/ou le réglage initial sont déterminés.

7. Procédé selon la revendication précédente, dans lequel l'étape d'étalonnage initial est réalisée pendant et/ou consécutivement à au moins une analyse de contrôle qualité du dispositif de chromatographie liquide-spectrométrie de masse (111) et/ou pendant et/ou consécutivement à au moins une analyse d'échantillons d'étalons internes.

8. Procédé selon l'une quelconque des deux revendications précédentes, dans lequel un début de l'étape d'étalonnage initial est déclenché par le changement d'une colonne du dispositif de chromatographie liquide-spectrométrie de masse (111) et/ou après un temps prédéfini ou prédéterminé et/ou après un nombre prédéfini ou prédéterminé d'analyses, ou d'autres compteurs appropriés.

9. Procédé selon l'une quelconque des revendications précédentes, dans lequel le réglage initial comprend un intervalle temporel large de temps de rétention par rapport au réglage réel.

10. Procédé selon l'une quelconque des revendications précédentes, dans lequel le procédé comprend la répétition des étapes a) à d) du procédé, dans lequel une largeur de la fenêtre de mesure (120) se rétrécit avec le nombre de répétitions.

11. Procédé selon l'une quelconque des revendications précédentes, dans lequel les mesures de référence et la mesure de la transition de l'analyte sont réalisées essentiellement dans les mêmes conditions.

12. Procédé selon l'une quelconque des revendications précédentes, dans lequel les étapes b) à c) du procédé et dans l'étape d) la détermination de l'information de pic mesuré de la transition de l'analyte sont réalisées par au moins un ordinateur.

13. Dispositif (110) de surveillance de transitions multiples d'au moins un analyte dans un échantillon comprenant :
- au moins un dispositif de chromatographie liquide-spectrométrie de masse (111) configuré pour la surveillance de transitions multiples ;
- au moins une base de données (112) configurée pour le stockage d'au moins un ensemble de données (114) comprenant au moins une mesure de référence (116) d'au moins une transition d'au moins un analyte ;
- au moins un dispositif d'évaluation (148), le dispositif d'évaluation (148) étant configuré pour la détermination d'au moins une information de pic de référence de la transition de l'analyte en utilisant un réglage initial d'une fenêtre de mesure (120), la fenêtre de mesure (120) étant définie par un intervalle temporel de temps de rétention, le dispositif d'évaluation (148) étant configuré pour la détermination d'un réglage réel de la fenêtre de mesure (120) prenant en compte l'information de pic de référence, la détermination comprenant l'ajustement de l'intervalle temporel, le réglage réel étant un réglage de la fenêtre de mesure (120) déterminé en prenant en compte l'information de pic de référence déterminée, le dispositif d'évaluation (148) étant configuré pour la détermination d'une information de pic mesuré de la transition de l'analyte d'une mesure consécutive de la transition de l'analyte avec le dispositif de chromatographie liquide-spectrométrie de masse (111) en utilisant le réglage réel de la fenêtre de mesure (120), ledit dispositif étant **caractérisé en ce qu'**une valeur moyenne et/ou une moyenne mobile d'une pluralité de mesures précédentes est utilisée comme mesure de référence (116).

14. Dispositif (110) selon la revendication précédente, dans lequel le dispositif (110) est configuré pour réaliser le procédé selon l'une quelconque des revendications précédentes faisant référence à un procédé.
